# EUROPEAN PATENT SPECIFICATION

(11) **EP 0 459 975 B1**
(45) Date of publication and mention of the grant of the patent: **13.12.1995**
(21) Application number: 89903922.6
(22) Date of filing: 06.03.1989
(51) Int. Cl.: A01N 63/04, C12N 1/14

(54) **BIOLOGICAL CONTROL OF WHITEFLIES AND OTHER PESTS WITH A FUNGAL PATHOGEN**
BIOLOGISCHE KONTROLLE VON WEISSEN FLIEGEN UND ANDEREN SCHÄDLINGEN MIT HILFE EINES PILZPATHOGENS
LUTTE A L'AIDE DE MOYENS BIOLOGIQUES CONTRE LE BEMISIA TABACI (GENNADIUS) ET D'AUTRES PARASITES EN UTILISANT UN PATHOGENE FONGIQUE

(43) Date of publication of application: 11.12.1991
(73) Proprietor: UNIVERSITY OF FLORIDA RESEARCH FOUNDATION, INC., Alachua, Florida 32615 (US)
(72) Inventor: OSBORNE, Lance, S., Longwood, FL 32779 (US)
(74) Representative: Perry, Robert Edward
(86) International application number: US8900910
(87) International publication number: WO9010388

(56) References cited:
- Biological Abstracts, Vol. 79, No. 8, 1985, N.K. MANIANIA et al.: "Host Specificity of Entomopathogenic Hyphomycetes to Noctuids", see pages AB-275, AB-276- Abstract No. 66630, & Entomophaga 29(4): 451-464, 1984
- Biological Abstracts, Vol. 70, No. 11, 1980, S.S. WASTI et al.: "Gypsy Moth (Lymantria Dispar) Mycoses by 2 Species of Entomogenous Fungi and an Assesment of their Avian Toxicity", see page 7377 Abstract No. 70587, & Parasitology 80(3): 419-424, 1980
- Biological Abstracts, Vol. 69, No. 2, 1980, G.C. HARTMANN et al.: "Murine Safety of 2 Species of Entomogenous Fungi Cordyceps Militaris and Paecilomyces Fumoso Roseus", see page 1115 Abstract No. 10514, & Appl. Entomol. Zool 14(2): 217-220, 1979
- Biological Abstracts, Vol. 68, No. 5, 1979, C. BAJAN et al.: "Utilization of Parasitic Microorganisms to Decrease Colorado Beetle Quantity", see page 2748 Abstract No. 27560, & Bull. Acad. Pol. Sci. Ser. Sci. Biol. 26(10): 715- 718, 1978
- Database CAB, File Supplier CAB International, Quest Accession Number 81642687, (Wallington, GB), "CMI Descriptions of Pathogenic Fungi and Bacteria", & Vol. Set 62, No. 611-620, 1979 (Commomwealth Mycological Institute)

## Description

### Background of the Invention

Whiteflies, mites, aphids, thrips, mealybugs, and other pests cause millions of dollars of damage each year to ornamental plants and plants grown in greenhouses. For example, the sweetpotato whitefly Bemisia tabaci (Gennadius) has appeared on poinsettias in California, Florida, Georgia, and North Carolina. During 1981, B. tabaci was responsible for crop and market losses of $100 million in cotton, cucurbits, and lettuce in California and Arizona. The whitefly is increasingly a problem in Florida where, in 1986, B. tabaci caused approximately $2 million of damage to Florida's $8 to 10 million poinsettia crop.

B. tabaci is also a pest of international importance, having been found on host plants throughout the mid-east Caribbean and Central America. This insect is now known to feed on more than 500 different plants, many of which are of importance in the Caribbean and Florida. For example, cassava, sweet potato, squash, tomato, beans, lettuce, cotton, pepper, carrot, cucumber, egg plant, and watermelon are all known hosts. This species of whitefly severely impacts infested plants by its feeding, production of honeydew with resultant growth of sooty mold, and transmission of plant pathogens. Most extensive losses to this pest have been through direct feeding damage and indirect damage through transmission of plant diseases.

Whitefly borne diseases are of major importance in tropical and subtropical agriculture. More than 70 diseases caused by viruses and microorganisms are known to be transmitted by whiteflies, with most of them being transmitted by B. tabaci. In Puerto Rico, this whitefly is a vector of at least seven diseases. One of these diseases is the bean golden mosaic virus, a disease affecting many legumes.

B. tabaci (Gennadius) has proven to be very difficult to control with conventional pesticide applications. Many factors contribute to the lack of control obtained with pesticides. The most important factor is that this whitefly has demonstrated a broad spectrum of resistance to chlorinated hydrocarbon, organophosphorus, carbamate, and synthetic pyrethroid insecticides. Very few commercially available pesticides are effective against whiteflies, and those which do work are only effective if care is taken to make a very thorough application of the insecticide several times a week. The sweetpotato whitefly spends most of its life on the undersides of leaves, therefore, growers must adjust their management practices to permit increased pesticide coverage there. The spacing of the plants must be such that the chemical spray can penetrate the canopy and reach all surfaces of the plants.

In addition to being largely ineffective, and difficult and costly to apply, chemical control of these pests has other significant drawbacks. For example, the use of chemical pesticides presents the further disadvantages of polluting the environment, creating potential health hazards to agricultural workers and to consumers, development of resistance to chemicals in target pest species, detrimental effect of these chemicals on non-target species resulting in secondary pest outbreaks, and phytotoxic reactions by treated plants.

Because of the problems associated with the use of chemical pesticides, safer and more effective methods of control for pests are clearly needed. Although biological control agents are actively being sought-after, to date no biological control agent has been commercially successful for the control of this whitefly.

Biological control agents are needed not only for B. tabaci, but also for other common pests of greenhouse and ornamental plants. These other common pest include mites, thrips, mealybugs, aphids, and scales. Twospotted spider mites, for example, feed on many species of plants and are a major pest of vegetables, ornamentals, fruit trees, hops, cotton, and strawberries. It is believed that widespread use of broad-spectrum insecticides destroy or greatly hamper natural enemies of spider mites and may thereby allow pest outbreaks to occur.

Biological control agents have been tried for many, if not all, of these pests. However, availability, limited host range, cost and reliability have reduced the potential for implementing the use of these biological control agents. The development of broad spectrum mycoinsecticides will reduce the need for many of the petrochemically based pesticides. By using fungi to control pests, the potential for resistance development is minimized, which, in turn, will stabilize many of the pest management programs.

Maniana et al, Biol. Abs. (1985) 79(8):AB275-276, disclose the activity of several fungal isolates, including 10 P. fumosoroseus isolates, against 4 noctuid species. No source or identification of the isolates is provided. Pathogenic activity was highly variable.

Wasti et al, Biol. Abs. (1980) 70(11):7377, Abstract No. 70587, report mycoses of gypsy moths by an isolate of Metarrhizium anisopliae and an isolate of P. fumosoroseus.

Database CAB, File Supplier CAB International, Quest Accession No. 81642687 (Wallingford, United Kingdom), states that P. fumosoroseus can be found in a wide range of insect hosts.

### Summary of the Invention

The present invention provides a novel, highly virulent Paecilomyces fumosoroseus and its use to control pests which attack ornamental plants and plants grown in greenhouses. Specifically exemplified herein is P. fumosoroseus Apopka. This fungus, advantageously, shows virulence against whiteflies, mites, aphids, thrips, mealybugs, and other pests. Its use does not produce the environmental hazards associated with chemical control agents. The fungus can be applied directly to the insects or it can be applied to the plants which are to be protected. The fungus can be used in greenhouses, nurseries, or any other place where pests are a problem. The present invention also provides mutants of P. fumosoroseus Apopka which substantially retain the virulence of the parent strain against nursery and greenhouse pests.

### Detailed Description of the Invention

A biologically pure culture of an isolate of the novel Paecilomyces fumosoroseus, designated Apopka, was deposited under the terms of the Budapest Treaty on 4th February 1988 in the American Type Culture Collection (ATCC), 12301 Parklawn Drive, Rockville, MD 20852, USA. The Accession Number is ATCC 20874.

The taxonomic description of the novel isolate of Paecilomyces fumosoroseus claimed here is the same as that for other members of that species. See Samson (Samson, R.A. [1974] "Paecilomyces and some allied Hyphomyces," Stud. Mycol. 6:1-116) for a taxonomic description of P. fumosoroseus. The novel isolate claimed here, Paecilomyces fumosoroseus Apopka, differs from other members of that species metabolically and biochemically in such a way that it is virulent against a number of important pests, as described below.

The novel fungus of the subject invention has been successfully grown on several different media including potato dextrose agar (PDA), V-8™ juice agar, lima bean agar, oatmeal agar, and mixed cereal agar. Based on the diameter of the colonies, the spore production, and the cost and availability of the agars, Difco PDA™ and V-8™ provide the best mediums for growing the fungus of the subject invention. Colonies on PDA are very fast growing with multinuclear colonies developing on the same plate shortly after the first sporulation occurs. A diameter of 4 cm. is obtained within 14 days at 24 C.

When whiteflies or other pests are exposed to Paecilomyces fumosoroseus the insects are killed when the fungus colonizes the insect. The fungus has been shown to colonize all life stages of the target insects thereby facilitating immediate and long-term control of the pests. Table 1 shows the results of experiments accessing the fungus' ability to infect the pupae of B. tabaci. The treatment group involved in these experiments was treated with Paecilomyces fumosoroseus Apopka at a concentration of 1 x 10⁶ spores/ml. The control group was not treated with the fungus and were dipped in a 1.2 g/500ml CAPTAN™ (Orthocide) solution to kill all contaminants.

**Table 1**

| Infection rate of 50 papae of B. tabaci after treatment with 1 x 10⁶ spores/ml solution of Paecilomyces fumosoroseus Apopka. | | |
|---|---|---|
| Days post-treatment | % infection | |
| | control | treatment |
| 3 | 0 | 64 |
| 5 | 0 | 76 |
| 6 | 0 | 82 |
| 7 | 0 | 86 |

Results of experiments assessing the fungus' ability to colonize the larvae of 2 species of whitefly--Bemisia tabaci (sweet potato whitefly) and Dialeurodes citri (citrus whitefly)--are shown in Table 2.

**Table 2**

| Colonization of Sweet Potato Whitefly, Bemisia tabaci and Citrus Whitefly, Dialeurodes citri by the novel fungus Paecilomyces fumosoroseus Apopka. | | |
|---|---|---|
| Concentration (# spores/ml) colonized | % of Bemisia tabaci colonized | % of Dialeurodes citri |
| --none | 0 | 0 |
| 1 x 10⁶ | 39 | 27 |
| 5 x 10⁶ | 41 | 38 |
| 1 x 10⁷ | 73 | 50 |

In addition to whiteflies, the fungus claimed here has also been observed to have activity against Thrips tabaci (onion thrips), Tetranychus urticae (Twospotted spider mite), Frankliniella (flower thrips), Echinothrips americanus.

Advantageously, the fungus of the claimed invention is not adversely affected by most chemical control agents. As Table 3 shows, very few of the commonly used chemical control agents show activity against the fungus. This means that the fungus can be used before, after, or in conjunction with chemical control agents. The chemical pesticides listed in Table 3 are all trade names known to persons in the pesticide art.

**Table 3**

| Activity of common chemical pesticides against the fungus Paecilomyces fumosoroseus. | |
|---|---|
| Chemical pesticide | Activity against P. fumosoroseus Apopka |
| Aliette 80% WP | NO |
| Avid | NO |
| Bayleton 25% WP | NO |
| Benlate 50% WP | NO |
| Botran 75W | NO |
| Carbamate 76% | NO |
| Chipco 26019 (50 WP) | NO |
| Cyprex 65% WP | NO |
| Daconil 2787 75% WP | NO |
| Dursban 50W | NO |
| Flutolanil | NO |
| Kocide 101 (77%) | NO |
| Lesan (35%) | NO |
| Manzate 200 (80%) WP | YES |
| Ornalin 50 WP | NO |
| Orthocide 50W | YES |
| Orthene | NO |
| Prochloraz | YES |
| Spotless | NO |
| Subdue 2E | NO |
| Sufers Insecticidal Soap | NO |
| Sulfur | NO |
| Talstan 10 WP | NO |
| Terraclor 75% WP | NO |
| Terraguard | YES |
| Truban 30% WP | NO |
| Vitavax | NO |
| Vydate 2L | NO |
| Zineb | NO |
| Zyban 75% WP | NO |

Spraying is the preferred method of applying the fungus of the claimed invention. The fungus may be applied to insects directly, to the foliage of plants, or the surroundings. A fungal suspension containing from about 1 x 10⁵ to about 1 x 10⁹ spores per milliliter of water and 2 drops of TWEEN™ per liter can be sprayed either on the insect or on the plants which are to be protected. The TWEEN™ acts as a wetting agent and other equivalent wetting agents can be used. In order to prepare the fungus for application, the spores can be harvested from a culture by pouring 0.05% TRITON™ in the petri dish and then diluting with sterile deionized water.

The fungus may also be applied in conjunction with a powder or granular carrier. As with spray application, the powder or granular formulation may be applied directly to the insect, the foliage of the plants, or the surroundings. To prepare the fungus for mixing with the powder or granular carrier it may be scraped or otherwise removed from the surface of the growth medium and combined with rice or any other granular or powder material which does not inhibit the growth of the fungus. Although the fungus may be applied in conjunction with a granular carrier, application may be easier and more uniform if the carrier and fungus mixture has a powder consistency. If necessary to achieve a particle size appropriate for easy application, the fungus/particle mixture may be slowly ground until the desired consistency is achieved. This means of formulation results in the application of a mixture which comprises fungal spores and mycelia together with a carrier. The presence of both the spores and the mycelia facilitates rapid and widespread colonization of the target insects. The application of the fungus and powder carrier can be accomplished using an aerosol applicator.

## Claims

1. A fungus of the species Paecilomyces fumosoroseus, characterised by being virulent against a pest selected from Bemisia tabaci, Dialeurodes citri, Thrips tabaci, Tetranychus urticae, Frankliniella sp, and Echinothrips americanus.

2. A fungus according to claim 1, wherein the pest is Bemisia tabaci.

3. A fungus according to claim 1 or claim 2, having the identifying characteristics of Paecilomyces fumosoroseus Apopka as available under the deposit ATCC 20874, or a mutant thereof.

4. A composition comprising a fungus according to any preceding claim, in association with an agricultural carrier.

5. A composition according to claim 4, wherein the carrier is particulate, e.g. a powder or granules.

6. A composition according to claim 4 or claim 5, wherein the carrier comprises rice or ground-up rice.

7. A composition according to claim 6, wherein the fungus is in the form of spores and/or mycelia.

8. A composition according to claim 4, wherein the carrier is liquid.

9. A composition according to claim 8, wherein the liquid comprises water and a wetting agent.

10. A composition according to claim 8 or claim 9, wherein the fungus is in spore form, at a concentration of 1 x 10⁵ to 1 x 10⁹ spores/ml of carrier.

11. A process for controlling a pest as defined in claim 1 or claim 2, which comprises applying a fungus according to any of claims 1 to 3 or a composition according to any of claims 4 to 10, to the pest or the foliage of plants, or to their surroundings.

12. A process according to claim 11, wherein a composition according to any of claims 8 to 10 is applied directly to individual pests or to small groups of pests.

13. A process according to claim 11, wherein a composition according to any of claims 5 to 7 is applied directly to the foliage of plants.

14. A process according to claim 11, wherein a composition according to any of claims 8 to 10 is applied directly to the foliage of plants.

15. A process according to any of claims 11 to 14, wherein the pest is whiteflies.

16. A process according to any of claims 11 to 14, wherein the pest is thrips.

17. A process according to any of claims 11 to 14, wherein the pest is aphids.

18. A process according to any of claims 11 to 14, wherein the pest is spider mites.

## Patentansprüche

1. Pilz der Spezies Paecilomyces fumosoroseus, gekennzeichnet durch seine Virulenz gegen einen Schädling, der aus Bemisia tabaci, Dialeurodes citri, Thrips tabaci, Tetranychus urticae, Frankliniella sp. und Echinothrips americanus ausgewählt ist.

2. Pilz nach Anspruch 1, wobei der Schädling Bemisia tabaci ist.

3. Pilz nach Anspruch 1 oder nach Anspruch 2 mit den identifizierenden charakteristischen Merkmalen von Paecilomyces fumosoroseus Apopka, wie erhältlich unter der Hinterlegung ATCC 20874, oder eine Mutante davon.

4. Zusammensetzung, die einen Pilz nach irgendeinem der vorhergehenden Ansprüche in Verbindung mit einem landwirtschaftlichen Träger umfaßt.

5. Zusammensetzung nach Anspruch 4, worin der Träger teilchenförmig ist, z.B. ein Pulver oder Granulat.

6. Zusammensetzung nach Anspruch 4 oder Anspruch 5, worin der Träger Reis oder gemahlenen Reis umfaßt.

7. Zusammensetzung nach Anspruch 6, worin der Pilz in Form von Sporen und/oder Myzelien vorliegt.

8. Zusammensetzung nach Anspruch 4, worin der Träger flüssig ist.

9. Zusammensetzung nach Anspruch 8, worin die Flüssigkeit Wasser und ein Benetzungsmittel umfaßt.

10. Zusammensetzung nach Anspruch 8 oder Anspruch 9, worin der Pilz in Sporenform mit einer Konzentration von 1 x 10⁵ bis 1 x 10⁹ Sporen/ml Träger vorliegt.

11. Verfahren zur Bekämpfung eines Schädlings wie in Anspruch 1 oder Anspruch 2 definiert, welches umfaßt, daß ein Pilz nach irgendeinem der Ansprüche 1 bis 3 oder eine Zusammensetzung nach irgendeinem der Ansprüche 4 bis 10 auf den Schädling oder das Blattwerk von Pflanzen oder deren Umgebung aufgebracht wird.

12. Verfahren nach Anspruch 11, worin eine Zusammensetzung nach irgendeinem der Ansprüche 8 bis 10 direkt auf einzelne Schädlinge oder kleine Gruppen von Schädlingen aufgebracht wird.

13. Verfahren nach Anspruch 11, worin eine Zusammensetzung nach irgendeinem der Ansprüche 5 bis 7 direkt auf das Blattwerk von Pflanzen aufgebracht wird.

14. Verfahren nach Anspruch 11, worin eine Zusammensetzung nach irgendeinem der Ansprüche 8 bis 10 direkt auf das Blattwerk von Pflanzen aufgebracht wird.

15. Verfahren nach irgendeinem der Ansprüche 11 bis 14, worin die Schädlinge Weißfliegen ("whiteflies") sind.

16. Verfahren nach irgendeinem der Ansprüche 11 bis 14, worin die Schädlinge Thrips sind.

17. Verfahren nach irgendeinem der Ansprüche 11 bis 14, worin die Schädlinge Blattläuse sind.

18. Verfahren nach irgendeinem der Ansprüche 11 bis 14, worin die Schädlinge Spinnenmilben sind.

## Revendications

1. Champignon de l'espèce Paecilomyces fumosoroseus, caractérisé en ce qu'il est virulent vis-à-vis d'un parasite sélectionné parmi Bemisia tabaci, Dialeurodes citri, Thrips tabaci, Tetranychus urticae, Frankliniella sp et Echinothrips americanus.

2. Champignon selon la revendication 1, dans lequel le parasite est Bemisia tabaci.

3. Champignon selon la revendication 1 ou 2, ayant les caractéristiques identificatrices de Paecilomyces fumosoroseus Apopka, tel que disponible sous le numéro ATCC 20874, ou un mutant de ce dernier.

4. Composition comprenant un champignon selon l'une quelconque des revendications précédentes, en combinaison avec un véhicule agricole.

5. Composition selon la revendication 4, dans laquelle le véhicule est particulaire, par exemple une poudre ou des granules.

6. Composition selon la revendication 4 ou 5, dans laquelle le véhicule comprend du riz ou du riz broyé.

7. Composition selon la revendication 6, dans laquelle le champignon est sous la forme de spores et/ou de mycéliums.

8. Composition selon la revendication 4, dans laquelle le véhicule est liquide.

9. Composition selon la revendication 8, dans laquelle le liquide comprend de l'eau et un agent mouillant.

10. Composition selon la revendication 8 ou 9, dans laquelle le champignon est sous la forme de spores, à une concentration de 1 x 10⁵ à 1 x 10⁹ spores/ml de véhicule.

11. Procédé de lutte contre un parasite selon la revendication 1 ou 2, lequel comprend l'application d'un champignon selon l'une quelconque des revendications 1 à 3 ou d'une composition selon l'une quelconque des revendications 4 à 10 sur le parasite ou le feuillage des végétaux ou leur environnement.

12. Procédé selon la revendication 11, dans lequel une composition selon l'une quelconque des revendications 8 à 10 est appliquée directement aux différents parasites ou à de petits groupes de parasites.

13. Procédé selon la revendication 11, dans lequel une composition selon l'une quelconque des revendications 5 à 7 est appliquée directement sur le feuillage des végétaux.

14. Procédé selon la revendication 11, dans lequel une composition selon l'une quelconque des revendications 8 à 10 est appliquée directement sur le feuillage des végétaux.

15. Procédé selon l'une quelconque des revendications 11 à 14, dans lequel le parasite est un aleurode.

16. Procédé selon l'une quelconque des revendications 11 à 14, dans lequel le parasite est un thrips.

17. Procédé selon l'une quelconque des revendications 11 à 14, dans lequel le parasite est un puceron.

18. Procédé selon l'une quelconque des revendications 11 à 14, dans lequel le parasite est un acarien jaune commun.
